# EUROPEAN PATENT APPLICATION

(11) **EP 2 277 576 A1**
(43) Date of publication of application: **26.01.2011**
(21) Application number: 09166330.2
(22) Date of filing: 24.07.2009
(51) Int. Cl.: A61M 15/00, B05B 17/06, A61M 11/00

(54) **Inhaler**

(71) Applicant: Vectura Delivery Devices Limited, Chippenham, Wiltshire SN14 6FH (GB); Boehringer Ingelheim International GmbH, 55216 Ingelheim am Rhein (DE)
(72) Inventor: Krakowka, Manuel, 55216 Ingelheim am Rhein (DE); Rohrschneider, Marc, 55216 Ingelheim am Rhein (DE); Wachtel, Herbert, 55216 Ingelheim am Rhein (DE); Sarkar, Matthew Neil, Cambridge CB4 3JU (GB); Eason, Stephen William, Norfolk IP22 1RX (GB); Swanbury, Philip John, Cambridge CB24 8PR (GB)
(74) Representative: Grey, Ian Michael

(57) **Abstract**

An inhaler (1) is proposed which comprises an electric or electro-mechanic or magnetic vibrating device (19). The vibrating device may be attached to or integrated into the blister (3) containing an inhalation formulation (4). Thus, deagglomeration and generating of an aerosol cloud can be improved.

## Description

The present invention relates to an inhaler according to the preamble of claim 1.

The present invention relates to inhalers for delivery of a preferably powder-form inhalation formulation from a reservoir, in particular a blister with a blister pocket, containing the preferably premetered inhalation formulation.

The present invention relates in particular to passive inhalers, i.e. inhalers where the patient or user breathes in to generate an air stream, which entrains the inhalation formulation and forms the desired aerosol.

It is difficult to achieve optimized discharge characteristics, in particular good de-agglomeration of the powdered inhalation formulation, and/or to generate an aerosol cloud with only fine particles, preferably in the range of 2 to 10 µm, in particular 2 to 7 µm, of the inhalation formulation.

Object of the present invention is to provide an inhaler with optimized discharge characteristics.

The above object is achieved by an inhaler according to claim 1. Advantageous embodiments are subject of the subclaims.

According to a first aspect of the present invention, the inhaler comprises an electric or electro-mechanic or magnetic vibrating device, which is preferably associated to the reservoir with the inhalation formulation. This enables or ensures improved de-agglomeration or loosening or aerosol generation of the inhalation formulation - in particular, this supports loosening or de-agglomeration of the inhalation formulation or powder in the reservoir or blister pocket - and, thus, optimized discharge characteristics.

The vibrating device may also be provided at or integrated into - in particular partly or completely - the reservoir of the inhaler, preferably, in a blister. Therefore, according to a second aspect of the present invention, which can be realized independently, the invention is directed to a blister which comprises an electric or electro-mechanic or magnetic vibrating device or at least part thereof.

It is possible to cause the blister pocket containing the inhalation formulation and/or the lid or any other part or portion of the blister and/or any other component of the inhaler to vibrate by means of the vibrating device. Thus, optimized de-agglomeration or loosening or aerosol generation of the inhalation formulation can be achieved resulting in optimized discharge characteristics.

In the present invention, the term "blister" has to be understood preferably in the usual sense in particular such that a pre-formed base is covered by a lid. However, the term "blister" can also be understood in a broader sense to include other, preferably similar reservoirs, in particular with at least partly flexible or elastic or thin components or walls, according to the present invention.

Further aspects, features, properties and advantages of the present invention are described in the claims and the subsequent description of preferred embodiments, with reference to the drawing. There are shown in:
- Fig. 1: a schematic sectional view of the principle of an inhaler without mouthpiece cover;
- Fig. 2: a schematic sectional view of the slightly more realistic inhaler with closed mouthpiece cover;
- Fig. 3: a partial schematic sectional view of the inhaler according to a first embodiment of the present invention;
- Fig. 4: a schematic view of details and possible arrangements of a vibrating device of the inhaler;
- Fig. 5: a schematic view of a blister with an associated vibrating device according to a second embodiment of the present invention;
- Fig. 6: a schematic view of a blister with an associated vibrating device according to a third embodiment of the present invention;
- Fig. 7: a schematic view of a blister with an associated vibrating device according to a fourth embodiment of the present invention;
- Fig. 8: a schematic view of a blister with an associated vibrating device according to a fifth embodiment of the present invention;
- Fig. 9: a schematic view of a blister with an associated vibrating device according to a sixth embodiment of the present invention;
- Fig. 10: a schematic view of a backside of a blister with an associated vibrating device according to a seventh embodiment of the present invention;
- Fig. 11: another schematic view of the seventh embodiment according Fig. 10;
- Fig. 12: a schematic view of a blister with an associated vibrating device according to an eighth embodiment of the present invention;
- Fig. 13: a schematic view of a blister with an associated vibrating device according to a ninth embodiment of the present invention; and
- Fig. 14: a schematic view of a guide member of the inhaler with an associated vibrating device according to a tenth embodiment of the present invention.

In the Figures, the same reference numbers are used for identical or similar parts, even if a repeated description is omitted. In particular identical or corresponding advantages and properties then also result or may be achieved.

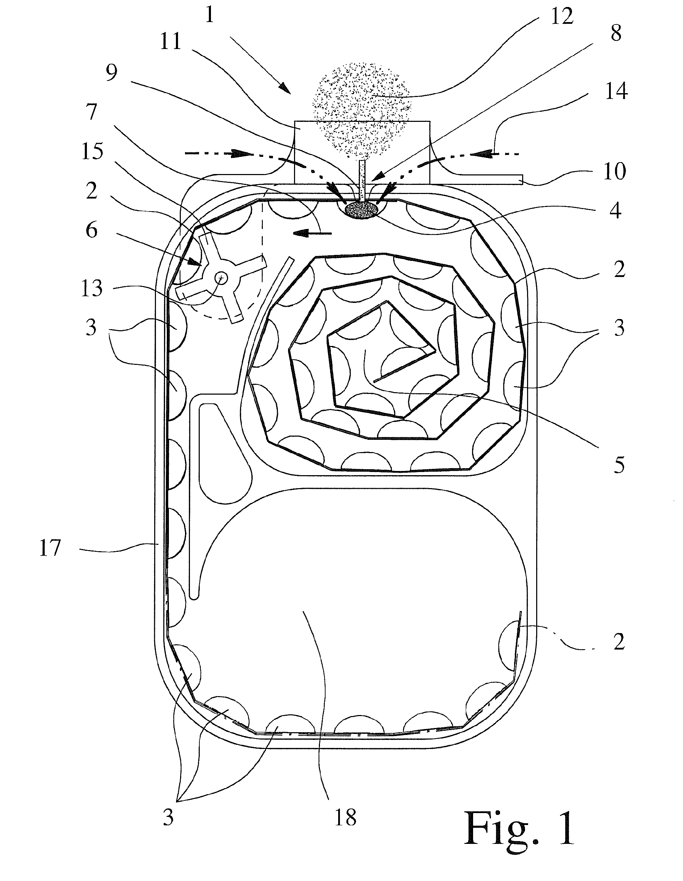

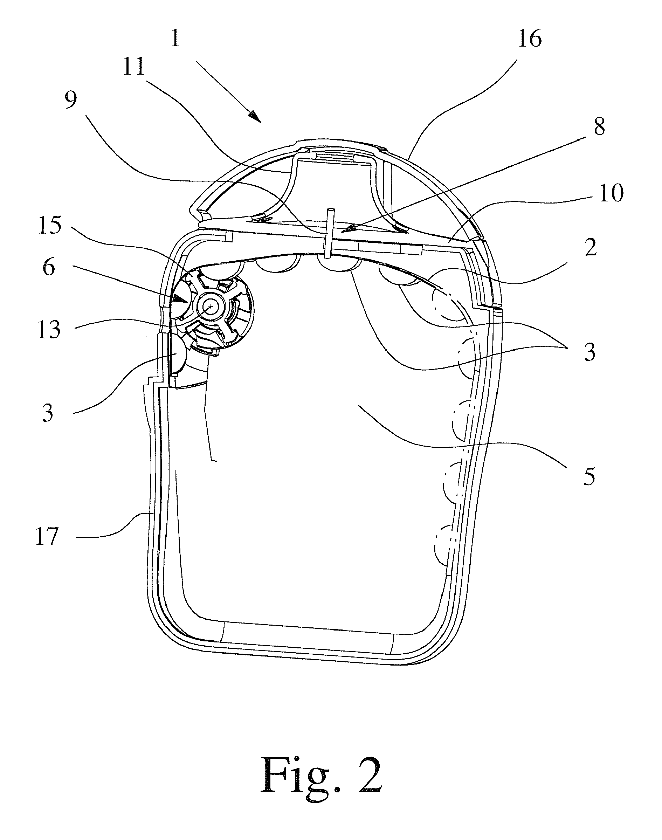

Fig. 1 and 2 show in very schematic sectional representations an inhaler 1.

The inhaler 1 serves to deliver a powdered inhalation formulation from a preferably band-shaped carrier, such as a blister strip 2. The blister strip 2 is preferably finite, i.e. not forming an endless or closed loop. The carrier has multiple receptacles, in particular blister pockets 3, respectively containing directly a dose of the loose inhalation formulation, in particular powder 4. Thus, the formulation is pre-metered in doses.

The inhaler 1 has a reservoir 5 for the still unused blister strip 2 with closed (sealed) receptacles. The unused blister strip 2 is preferably rolled up or wound up or stored in the reservoir 5.

The inhaler 1 has preferably a conveyor 6 for onward movement of the blister strip 2 in direction of arrow 7 stepwise by one blister pocket 3, in order to feed the blister pockets 3 successively (individually) to an opening position where the respective blister pocket 3 can be opened and emptied.

The inhaler 1 comprises a removal device 8, preferably a piercing member, for opening the receptacles individually (one after the other) and/or removing individually the does from the receptacles. In particular, the removal device 8 comprises a piercing element 9. The opening position of the respective blister pocket 3 is preferably adjacent to or below the removal device 8 or piercing element 9

The conveyor 6 and/or removal device 8 comprise a preferably lever-like actuating member 10. The actuating member 10 is pivotable around pivot axis 13 for driving or actuating the conveyor 6.

The actuating member 10 supports or holds the removal device 8 or piercing element 9. In particular, the actuating member 10 is pivotable or moveable such that the removal device 8 or its piercing element 9 can open or pierce a blister pocket 3 in the opening position.

In particular, the removal element / piercing element 9 punctures or cuts open a lid of the respective receptacle / blister pocket 3 that is in the opening position.

Preferably, the piercing element 9 is hollow and/or in fluid connection with a preferably adjacent mouthpiece 11 of the inhaler 1. Thus, the respective blister pocket 3 is not only opened, but preferably also fluidically connected to the mouthpiece 11 when the guide member 10 has moved into the removal position shown in Fig. 1 where the removal element extends into the respective blister pocket 3.

During or for inhalation a patient or user, not shown, places the mouthpiece 11 in his mouth and breathes in. An air stream 14 of ambient air is sucked in and passes through the opened blister pocket 3 such that the loose powder 4 (forming the inhalation formulation and being schematically shown in Fig. 1 only in the blister pocket 3 in the opening position) is dispensed with the sucked-in ambient air as an aerosol cloud 12 via the mouthpiece 11. This situation is schematically represented in Fig. 1. Thus, the respectively opened blister pocket 3 is emptied.

The mouthpiece 11 is preferably supported or mounted to the actuating member 10 and, thus, moveable together with the actuating member 10.

The actuating member 10 can be pivoted forth and back in order to drive the conveyor 6, in particular a conveying wheel 15 of the conveyor 6. Thus, the conveying wheel 15 can be rotated in one direction around pivot axis 13 in order to move the blister strip 2 in onward direction 7 stepwise, i.e. to index to the next blister pocket 3.

Preferably, the conveyor 6 comprises only one single conveying wheel 15, for advancing the carrier onwards, i.e. for moving the carrier in onward direction 7 and/or for positioning the respective receptacle / blister pocket 3 in the opening position. Preferably, the inhaler 1 and/or conveyor 6 do not comprise any further wheel. This allows a very compact and simple construction of the inhaler 1.

The conveying wheel 15 preferably engages between the blister pockets 3 and, thus, can convey the blister strip 2 in form-locking or form-fit manner. This allows a very secure and/or precise moving or advancing and, in particular, positioning of the blister strip 2.

The inhaler 1 comprises preferably a mouthpiece cover 16 associated to the mouthpiece 11. The mouthpiece cover 16 is pivotable between an open position (not shown) where the mouthpiece 11 is uncovered, and a closed position shown in figure 2 where the mouthpiece 11 is closed, and vice versa. The mouthpiece cover 16 is preferably pivotably mounted to or supported by the inhaler 1, in particular a housing 17 of the inhaler 1.

The used carrier, i.e. blister strip 2 with opened blister pockets 3, is preferably stored within the inhaler 1, in particular its housing 17. This used carrier may be stored in the reservoir 5 or in a separated space 18. Preferably, the used carrier is wound up within the inhaler 1.

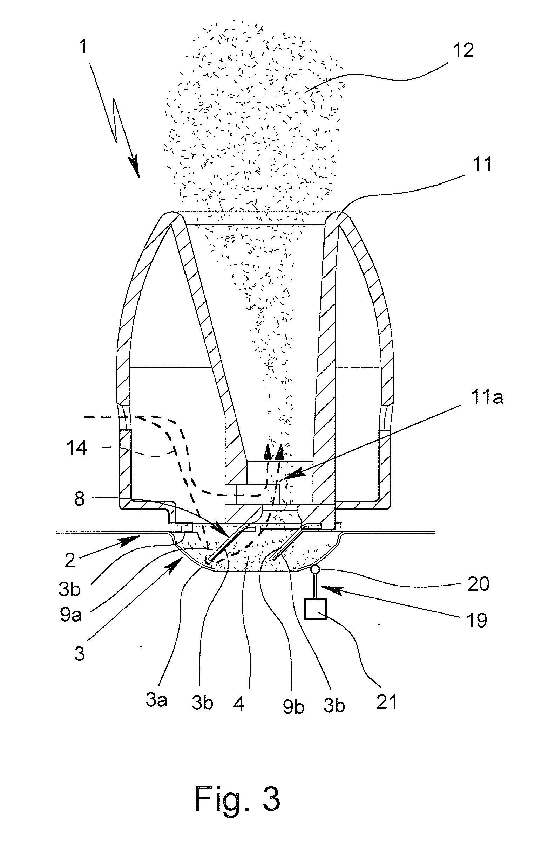

Fig. 3 shows in a schematic enlarged sectional view of the area of removal device 8 according to a first embodiment of the present invention. Fig. 3 shows the inhaler 1 with already opened blister pocket 3 during delivery (dispensing) of the inhalation formulation.

The blister strip 2 or blister pocket 3 preferably consists of or comprises a base 3a covered by a lid 3b. In particular, the base 3a forms or comprises at least one curved or bowl-like receiving space or depression covered by the lid 3b for sealingly receiving the inhalation formulation (powder 4). However, other designs are possible.

In the shown embodiment, the piercing member 8 preferably comprises at least one, here two piercing elements 9a, 9b for opening or puncturing the blister pocket 3, in particular its lid 3b as shown in Fig. 3.

In particular, the first piercing element 9a serves the form a first blister opening (inlet opening) in the lid 3b. The second piercing element 9b forms a separate, second blister opening (outlet opening) in the lid 3b.

The piercing member 8 or opened blister pocket 3 is in fluid connection or connectable with the feeding path 11a of the inhaler 1 to deliver or dispense the inhalation formulation via the mouthpiece 11 or the like.

During or for inhalation a patient or user, not shown, places the mouthpiece 11 in his mouth and breathes in. Thus, an air stream of ambient air is sucked in and passed or guided through the opened blister pocket 3 such that loose powder 4 is entrained in the air and dispensed with the sucked-in ambient air as an aerosol cloud 12 via the feeding path 11a and mouthpiece 11, as schematically shown Fig. 3.

In the present embodiment, preferably air is used to dispense the inhalation formulation. However, any other fluid or gas could be used. Preferably, the term "air" or "air stream" has to be understood in a broad sense including other gas as well.

In the present embodiment, the inhaler 1 is preferably a passive inhaler, i.e. the inhalation formulation is dispensed by breathing in of a patient or user (not shown). However, the present description and invention preferably also apply to active inhalers, i.e. when a means for pressurizing gas or any other fluid (e.g. a compressor, an air pump, liquefied or compressed gas or the like) is provided to actively dispense the inhalation formulation.

The present invention deals in particular with the dispensing of an inhalation formulation in powder-form by means of a gas stream. The following description focuses on this scenario. However, the present invention may also be used to generate an aerosol by means of any other fluid as medium for dispensing the inhalation formulation, e.g. a liquid dissolving and/or dispensing a dry inhalation formulation. Alternatively or additionally, a liquid inhalation formulation can be dispensed by means of gas and/or a liquid as feeding medium. Then, the present explanations shall apply preferably accordingly.

According to the present invention, an electric or electro-mechanic or magnetic vibrating device 19 is provided. The vibrating device 19 is in particular associated to the reservoir / blister 2 / blister pocket 3. In particular, the vibrating device 19 generates vibrations or hits so that at least part of the blister pocket 2, in particular at least a portion of the base 3a and/or lid 3b, and/or the feeding path 11a vibrate. This facilitates or supports or enables or ensures and improved de-agglomeration, loosening or aerosol generating of the inhalation formulation.

However, the vibrating device 19 can alternatively or additionally also cause the piercing member 8 or any other element extending into the preferably opened blister pocket 3 and/or any other component of the inhaler 1 to vibrate.

Fig. 3 shows only a very schematic view how the vibrating device 19 may be associated to the reservoir blisterstrip 2 or blister pocket 3.

The vibrating device 19 comprises preferably a contact, hammer or vibrating element 20 (hereinafter called vibrating element) and a drive 21 driving the vibrating element 20 as shown in Fig. 1. Here, the vibrating element 20 contacts the blister pocket 3 or its base 4 from outside.

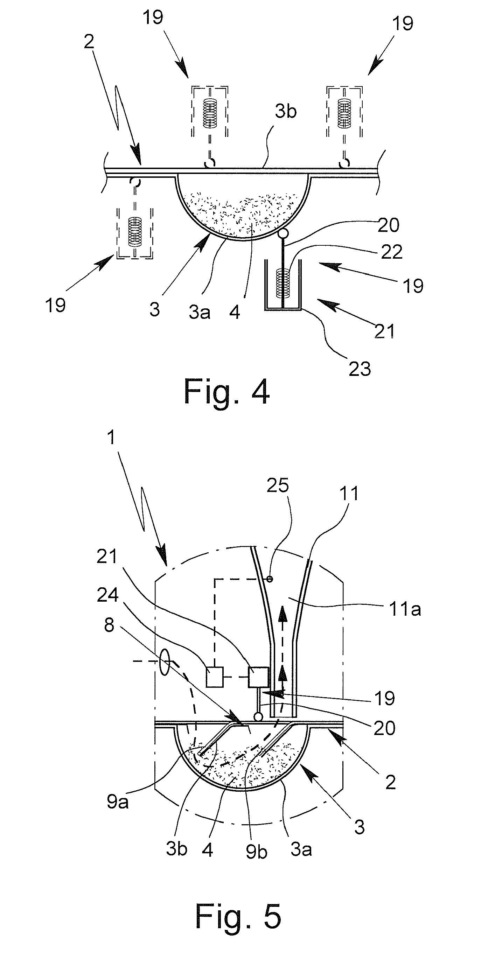

Fig. 4 shows in a very schematic view a preferred design of the vibrating device 19. In particular, the vibrating device 19 or drive 21 comprises a plunger coil or moving coil 22 that can oscillate relative to an associated, preferably bell-shaped magnet 23. Preferably the plunger coil 22 directly moves or actuates the vibrating element 20. This construction or other constructions, in particular as used in microphones, are particularly suitable to generate vibrations and/or to achieve the desired vibration frequencies as described later.

In the first embodiment, the vibrating device 19 contacts the blister 2, in particular the blister pocket 3, more precisely, the base 3a in the region of the blister pocket 3, from the outside. However, other arrangements are possible.

Fig. 4 shows in dashed lines other possible arrangements of the vibrating device 19. For example, the vibrating device 19 or its vibrating element 20 can contact the blister 2 from its backside, i.e. the base 3a, adjacent to the blister pocket 3.

The vibrating device 19 or vibrating element 20 can contact alternatively or additionally also the front side of the blister 2, i.e. the lid 3b, optionally in the area where the lid 3b covers the blister pocket 3, i.e. is not in contact with the base 3a, or where the lid 3b is in contact with the base 3a as shown in Fig. 4 by the two upper possible placements of the vibrating device 19.

Fig. 5 shows in a schematic view the inhaler 1 according to a second embodiment of the present invention. Here, the vibrating device 19 can cause at least part of the piercing member 8 to vibrate.

In the second embodiment, the inhaler 1 comprises preferably a control device 24, preferably an integrated circuit, a microcontroller or the like, for controlling the vibrating device 19. Most preferably, the vibrating device 19 is electrically or electronically controlled and/or operated. Preferably, the inhaler 1 and/or control device 24 are designed such that the operation of the vibrating device 19 can be controlled in different aspects, e.g. with regard to time, duration, intensity, amplitude, frequency or the like of the generated or caused vibration or hits.

Further a sensor 25 may be provided so that the vibration generation may be controlled also depending on additional information, such as air pressure in the mouthpiece 11, flow rate of inhalation air, actuation of a button or the like by the patient or user (not shown), or the like.

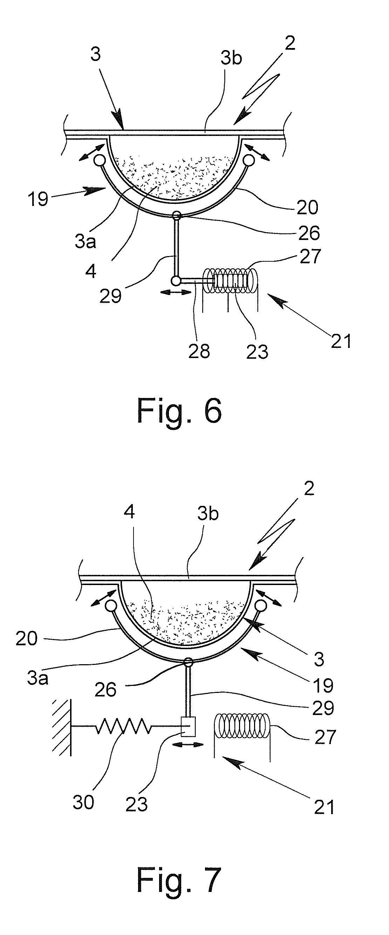

Fig. 6 shows in a schematic side view a third embodiment. Here, the vibrating device 19 or element 20 contacts or vibrates the blister pocket 3 at two different or opposite sides or portions and/or in two different or opposite directions. Preferably, the vibrating element 20 is a two-armed lever pivotable around an axis 26 in the third embodiment. For example, the drive 21 comprises a hollow coil 27 in which the magnet 23 or a magnetic element (anchor) can be electrically moved back and forth. This movement is transmitted in particular by means of a suitable drive train, e.g. an actuating element 28 connected with to magnet 23 or magnetic element and an actuating arm 29 pivotally connected with the actuating element 28 and radially connected with the vibrating element 20 as shown in Fig. 4.

Fig. 7 shows a fourth embodiment which is similar to the third embodiment. Here, the magnet 23 or a magnetic element (anchor) is moved by the electromagnet / coil 27 against the force of a spring 30 back and forth. Thus, a preferably perodic attraction of the magnet 23 / magnetic element is sufficient. This arrangement is particularly suitable for discrete hits to cause the blister 2 to or its base 3a to vibrate.

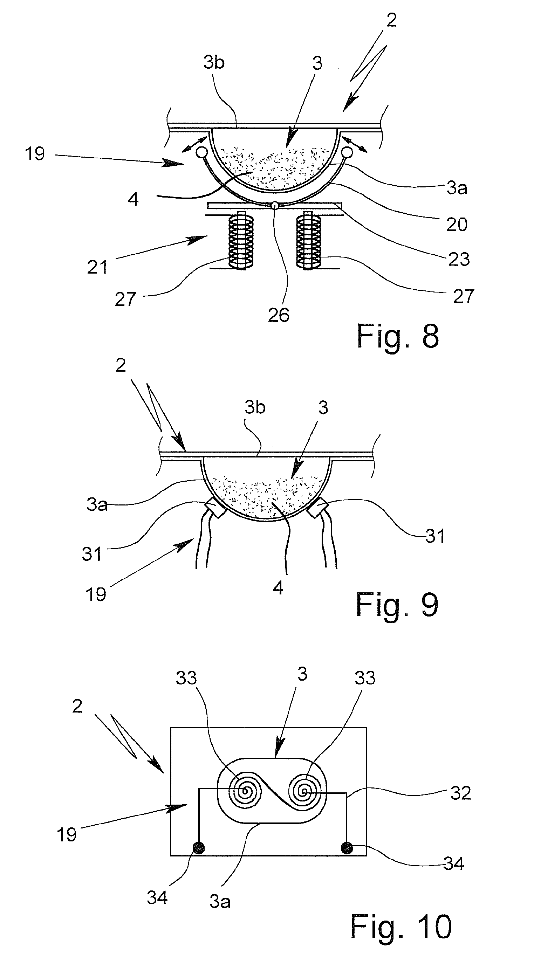

Fig. 8 shows a fifth embodiment, which is similar to the third and fourth embodiment. Here two electromagnets or coils 27 alternatively attract the preferably bar like magnet 23 or magnetic element / anchor so that the vibrating element 20 is pivoted back and forth.

Fig. 9 shows a sixth embodiment. Here, the vibrating device 19 comprises at least one, preferably two or more piezo elements 31 (preferably stacks of piezoelectric crystals or he like) that can cause the blister pocket 3, in particular its base 3a, to vibrate either indirectly - e.g. via a respective mechanic, drive train or the like (not shown) - or directly (by respective contact with the base 3a or any other part of the reservoir or any other part that shall vibrate). In particular, it is possible and provided that the piezo element(s) 31 is/are directly attached to or integrated into the part that shall vibrate, such as the blister 2, blister pocket 3, base 3a or lid 3b. In the shown embodiment, the piezo elements 31 are preferably located on opposite portions or sides of the blister pocket 3 or base 3a. However, other arrangements are also possible.

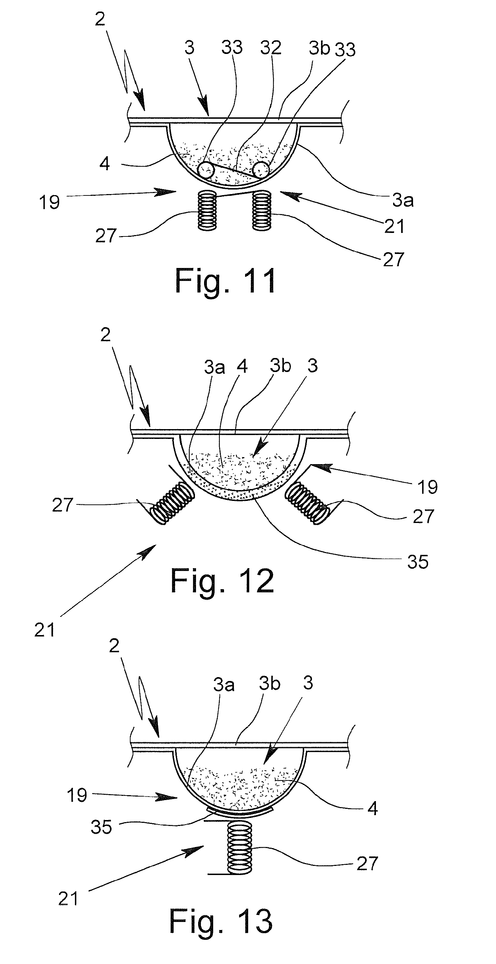

Fig. 10 and 11 show a seventh embodiment of the present invention. Here, a conductor 32 is formed directly on or integrated into the blister 2, in particular the base 3a in the area of the blister pocket 3.

The conductor 32 may form one or multiple coils 33 or other conducting arrangements. If necessary, the conductor 32 or blister 2 may comprise or form associated electric contacts 34 as shown in Fig. 10. The conductor 32 may be attached, e.g. glued, to the blister 2 or its base 3a or formed by etching or by any other suitable process.

The conductor 32 and/or coils 33 cooperate preferably with one or two preferably stationary electromagnets or coils 27 as shown in Fig. 11. By respectively controlling the current flowing through the conductor 32 / coil(s) 33 and through the coils 27, the blister pocket 3 or base 3a can be caused to vibrate, in particular if the preferred pairs of one coil 27 and one adjacent coil 33 attract alternatively or simultaneously.

It has to be noted that instead of the stationary coils 27 also stationary magnets or magnetic elements arranged near by the coils 33 could be used. Then, the movement and vibrating of the base 3a is only controlled by the electric current flowing through the conductor 32 / coil(s) 33.

Fig. 12 shows an eighth embodiment. Here, magnetic material 35 is attached to or integrated into the wall or part of portion, in particular base 3a, that shall vibrate. The magnetic material may be attracted by one or multiple electromagnets or coils 27 as schematically shown in Fig. 10. Thus, it is possible to vibrate the base 3a or any other flexible part with the magnetic material 35 as desired, e.g. by alternatively attracting portions with the magnetic material 35 or by periodically attracting portions with the magnetic material 35.

Alternatively the base 3a or portion, that shall vibrate, can be made of magnetic material.

Fig. 13 shows a ninth embodiment, which is very similar to the eighth embodiment. Here, the magnetic material 35 forms a coating or cover on the blister 2, in particular on the base 3a, or any other portion or component that shall vibrate.

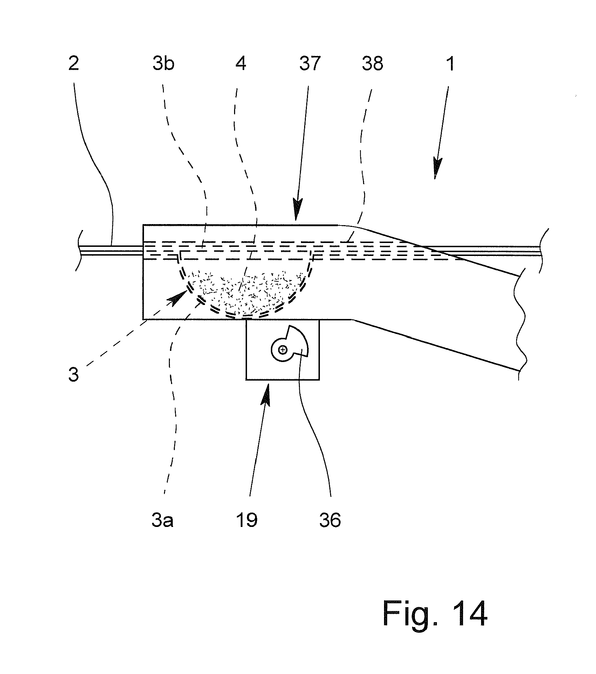

Fig. 14 shows a tenth embodiment. Here, the vibrating device 19 is associated or connected to aguide element 37 of the inhaler 1 to vibrate at least part of the guide element 37 and, thus part of the reservoir / blister strip 2. The guide element 37 may guide the blister strip 2 preferably in a form-fit manner, in particular in opposing grooves 38 in which the blister strip 2 slides. However, the vibrating device 19 can be associated or connected to any other component of the inhaler 1. In any case, the vibrating device 19 vibrates at least part of the blister strip 2 or respective blister pocket 3 directly or indirectly.

In the tenth embodiment, the vibrating device 19 comprises or forms an unbalance motor 36. The unbalance motor 36 is preferably electrically driven, particular by an accumulator or battery of the inhaler 1 not shown. However, the unbalance motor 36 could also be mechanically driven.

In general, the vibrating device 19 could also be connected or attached to the housing 17 or any other component of the inhaler 1, so that more or less the complete reservoir or blister strip 2 or more or less essentially all blister pockets 3 are caused to vibrate when the vibrating device 19 is operated and/or the inhaler 1 is used.

In general, the vibrating device 19 is used or operated preferably already before (or only before) the stream of fluid flows through the reservoir and/or before a user breathes in for inhaling. Thus, the powder 4 can be deagglomerated before the fluid (gas) entrains the powder 4 to discharge the respective dose of powder 4.

The inhaler 1 and/or vibrating device 19 may be designed such that the vibrating device 19 only assists the deagglomeration and aerosol forming by means of the fluid flowing through the reservoir.

In particular, the inhaler 1 and/or vibrating device 19 may be designed such that the inhaler 1 can deliver and discharge a dose of the inhalation formulation, in particular as aerosol cloud 12, even if the vibrating device 19 is not working or shut off. This may form a back up if the vibrating device 19 were defect.

In general, the frequency of vibration is preferably adapted to the respective component or part that shall vibrate.

In particular, the vibration frequency is adapted to the respective inhaler 1, respective reservoir, to the respective inhalation formulation, to the amount and/or consistence of the inhalation formulation and/or the like.

According to one preferred aspect, the vibration frequency of the vibrating device 19 or a harmonic frequency thereof corresponds essentially - at least temporarily - to the resonance frequency of the part or portion that shall vibrate, such as the blister 2, blister pocket 3, lid 3b, base 3a, piercing member 8, piercing element 9a or 9b, feeding path 11a and/or mouthpiece 11.

Most preferably, the vibration frequency of the vibrating device 19 is between 1 and 500 kHz, in particular about between 10 and 200 kHz.

According to another or additional aspect of the present invention, the vibration frequency of the vibrating device 19 varies, in particular wobbles, during a delivery operation or inhalation process.

The vibrating device 19 may operate feedback-controlled, e.g. as a hammer break or hammer interrupter.

Additionally or alternatively, the vibrating device 19 or its vibrating element 20 may act like a hammer that transmits one pulse or hit or multiple pulses or hits by respective strikes or hits to the respective component. Thus, the component will vibrate with its resonance frequency. The hammering frequency can be considerably lower, e.g. in the range of 5 Hz and 500 Hz.

It has to be noted that features of the different embodiments and/or the different embodiments may be combined as desired and/or used for other inhalers or reservoirs as well.

### List of reference numbers

- 1: inhaler
- 2: blister strip
- 3: blister pocket
- 3a: base
- 3b: lid
- 4: powder
- 5: reservoir
- 6: conveyor
- 7: onward direction
- 8: removal device
- 9: piercing element
- 9a: first piercing element
- 9b: second piercing element
- 10: actuating member
- 11: mouthpiece
- 11a: feeding path
- 12: aerosol cloud
- 13: pivot axis
- 14: air stream
- 15: conveying wheel
- 16: mouthpiece cover
- 17: housing
- 18: space
- 19: vibrating device
- 20: vibrating element
- 21: drive
- 22: plunger coil
- 23: magnet
- 24: control device
- 25: sensor
- 26: axis
- 27: stationary coil
- 28: actuating element
- 29: actuating arm
- 30: spring
- 31: piezo element
- 32: conductor
- 33: coil
- 34: contact
- 35: magnetic material
- 36: unbalance motor
- 37: guide element
- 38: groove

## Claims

1. Inhaler (1) for delivery of an inhalation formulation from a reservoir, preferably a blister (2) with a blister pocket (3), containing the inhalation formulation, comprising:
a mouthpiece (11); and
a feeding path (11a) fluidically connected or connectable to the reservoir;
the inhaler (1) being designed such that a stream of a fluid, in particular gas, preferably air, can discharge a dose of the inhalation formulation from the reservoir and through the feeding path (11a) to deliver it via the mouthpiece (11),
**characterized in that** the inhaler (1) or reservoir comprises an electric or electro-mechanic or magnetic vibrating device (19).

2. Inhaler according to claim 1, **characterized in that** the inhaler (1) is designed such that the vibrating device (19) can cause at least part of the reservoir to vibrate and/or at least part of the feeding path (11a) to vibrate.

3. Inhaler according to one of the previous claims, **characterized in that** the inhaler (1) is designed such that the vibrating device (19) can cause at least part of a piercing member (8) or any other element extending into the reservoir to vibrate.

4. Inhaler according to one of the previous claims, **characterized in that** the vibration frequency of the vibrating device (19) or a harmonic frequency thereof corresponds essentially - at least temporarily - to the resonance frequency of at least part of the reservoir, such as a base (3a) or lid (3b), and/or to the resonance frequency of at least part of a piercing member (8) or any other element extending into the reservoir.

5. Inhaler according to one of the previous claims, **characterized in that** the vibration frequency of the vibrating device (19) is between 1 and 500 kHz, preferably about between 10 and 200 kHz.

6. Inhaler according to one of the previous claims, **characterized in that** the inhaler (1) is designed such that the vibration frequency of the vibrating device (19) varies, in particular wobbles, during a delivery operation of the inhaler (1).

7. Inhaler according to one of the previous claims, **characterized in that** the vibrating device (19) or at least a part thereof, such as an electric coil (33) or a magnetic, electric or piezoelectric element (31, 32, 35), is attached to or integrated into the reservoir.

8. Inhaler according to one of the previous claims, **characterized in that** the vibrating device (19) comprises an electromagnet or a coil (27) or a plunger coil (22), and an associated magnet (23) or magnetic element or anchor.

9. Inhaler according to one of the previous claims, **characterized in that** the vibrating device (19) comprises a piezoelectric element (31).

10. Inhaler according to one of the previous claims, **characterized in that** the vibrating device (19) comprises a hammer or vibrating element (20) for contacting the reservoir preferably from the outside and/or on different portions or opposite sides to transmit a hit or vibration to the reservoir or any other component or portion of the inhaler (1) that shall vibrate.

11. Inhaler according to one of the previous claims, **characterized in that** the vibrating device (19) comprises or forms an unbalance motor (36).

12. Inhaler according to one of the previous claims, **characterized in that** the vibrating device (19) is used or operable before the stream of fluid flows through the reservoir and/or before a user breathes in for inhaling.

13. Inhaler according to one of the previous claims, **characterized in that** the vibrating device (19) only assists deagglomention of the inhalation formulation and aerosol forming by means of the fluid flowing through the reservoir, and/or that the inhaler (1) is designed to deliver a dose of the inhalation formulation, in particular as an aerosol (12), even if the vibrating device (19) is not working or shut off.

14. Inhaler according to one of the previous claims, **characterized in that** the inhaler (1) comprises a guide element (37) for guiding the moveable reservoir and is designed such that the vibrating device (19) can cause at least part of the guide element (37) to vibrate in order to vibrate at least part of the reservoir.

15. Inhaler according to one of the previous claims, **characterized in that** the inhalation formulation is powder (4).
